# EUROPEAN PATENT SPECIFICATION

(11) **EP 1 608 339 B1**
(45) Date of publication and mention of the grant of the patent: **21.03.2012**
(21) Application number: 03754445.9
(22) Date of filing: 04.09.2003
(51) Int. Cl.: A61K 31/415, A61K 31/455

(54) **PHARMACEUTICAL CO-CRYSTAL OF CELECOXIB-NICOTINAMIDE**
PHARMAZEUTISCHE MISCHKRISTALLE VON CELECOXIB-NICOTINAMID
COMPOSITIONS CO-CRISTALLINES DE CELECOXIB-NICOTINAMIDE

(30) Priority: 28.02.2003 US 451213 P; 03.03.2003 US 378956; 18.04.2003 US 463962 P; 11.07.2003 US 487064 P
(43) Date of publication of application: 28.12.2005
(73) Proprietor: McNeill-PPC, Inc., Skillman, NJ 08558 (US); UNIVERSITY OF SOUTH FLORIDA, Tampa, FL 33620-7900 (US); THE REGENTS OF THE UNIVERSITY OF MICHIGAN, Ann Arbor, Michigan 48109-1280 (US)
(72) Inventor: ALMARSSON, Örn, Shrewsbury, MA 01545 (US); BOURGHOL HICKEY, Magali, Medford, MA 02155 (US); PETERSON, Matthew, Framingham, MA 01701 (US); ZAWOROTKO, Michael, J., Tampa, FL 3362 (US); MOULTON, Brian, Providence, RI 02912 (US); RODRIGUEZ-HORNEDO, Nair, Ann Arbor, MI 48103 (US)
(74) Representative: Cole, Paul Gilbert
(86) International application number: PCT/US2003/027772
(87) International publication number: WO 2004/078161

(56) References cited:
- WO-A-01/42222
- WO-A-01/51919
- WO-A-03/033462
- WO-A-03/074474
- WEISSBUCH I ET AL: "Understanding and control of nucleation, growth, habit, dissolution and structure of two- and three-dimensional crystals using 'tailor-made' auxiliaries" 1995, ACTA CRYSTALLOGRAPHICA. SECTION B, STRUCTURAL SCIENCE, MUNKSGAARD, COPENHAGEN, DK, PAGE(S) 115-148 , XP002960386 ISSN: 0108-7681 the whole document abstract page 145, column 2, paragraph 2

## Description

The present invention relates to a co-crystal of celecoxib, pharmaceutical compositions comprising it, and methods for preparing the same.

Active pharmaceutical ingredients (API or APIs (plural)) in pharmaceutical compositions can be prepared in a variety of different forms. Such APIs can be prepared so as to have a variety of different chemical forms including chemical derivatives or salts. Such APIs can also be prepared to have different physical forms.

For example, the APIs may be amorphous, may have different crystalline polymorphs, or may exist in different salvation or hydration states. By varying the form of an API, it is possible to vary the physical properties thereof. For example, crystalline polymorphs typically have different solubilities from one another such that a more thermodynamically stable polymorph is less soluble than a less thermodynamically stable polymorph. Pharmaceutical polymorphs can also differ in properties such as shelf-life, bioavailability, morphology, vapour pressure, density, colour, and compressibility. Accordingly, variation of the crystalline state of an API is one of many ways in which to modulate the physical properties thereof.

It would be advantageous to have new forms of these APIs that have improved properties, in particular, as oral formulations. Specifically, it is desirable to identify improved forms of APIs that exhibit significantly improved properties including increased aqueous solubility and stability. Further, it is desirable to improve the processability, or preparation of pharmaceutical formulations. For example, needle- like crystal forms or habits of APIs can cause aggregation, even in compositions where the API is mixed with other substances, such that a non-uniform mixture is obtained. It is also desirable to increase the dissolution rate of API-containing pharmaceutical compositions in water, increase the bioavailability of orally administered compositions, and provide a more rapid onset to therapeutic effect. It is also desirable to have a form of the API which, when administered to a subject, reaches a peak plasma level faster, has a longer lasting therapeutic plasma concentration, and higher overall l exposure when compared to equivalent amounts of the API in its presently-known form.

WO 01/42222 (Pharmacia) discloses that a poorly water soluble selective cyclooxygenase-2 inhibitory compound such as celecoxib, deracoxib, valdecoxib or rofecoxib provides more rapid onset of therapeutic effect if, upon oral administration of a composition comprising the compound, the compound exhibits pharmacokinetic properties leading to a greater maximum blood serum concentration (Cmax) and/or a shorter time following the administration to reach that maximum (Tmax). A greater Cmax and/or a shorter Tjnax are obtained by reduction of size of solid particles comprising the compound such that a substantial portion by weight of the particles are smaller than 1 mm in diameter, in the longest dimension of the particles. The specification further discloses novel polymorphic forms of celecoxib.

It has now been found that new co-crystalline forms of APIs can be obtained which improve the properties of APIs as compared to such APIs in a non-co-crystalline state (free acid, free base, zwitterions, salts, etc.).

This invention relates to a co-crystal of celecoxib and nicotinamide, and to pharmaceutical compositions containing that co-crystal.

The invention will be described below with reference to the accompanying drawings, which are as follows:
Fig. 1 - Dissolution profile for a co-crystal of celecoxib : nicotinamide vs. celecoxib free acid.
Fig. 2 - Hygroscopicity profile for a co-crystal of celecoxib : nicotinamide vs. celecoxib sodium.

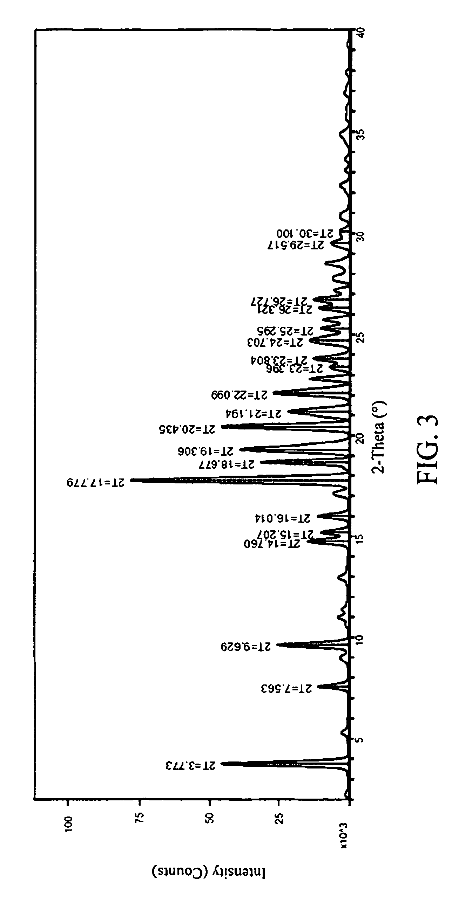

Fig. 3 - PXRD diffractogram for a co-crystal of celecoxib and nicotinamide.

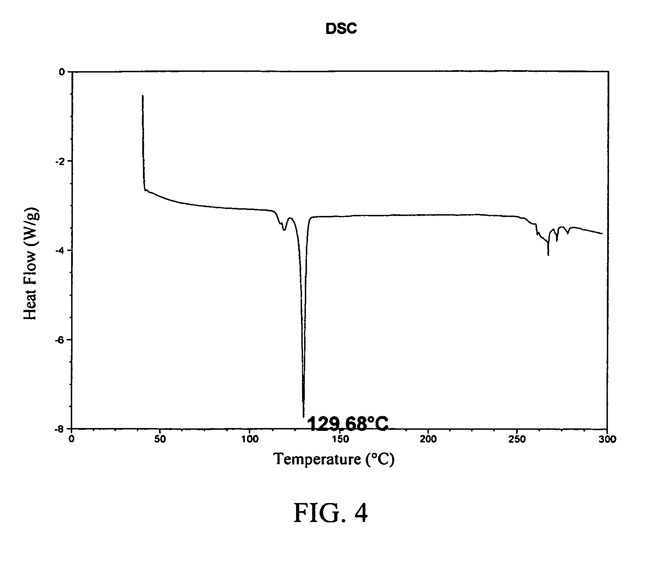

Fig. 4 - DSC thermogram for a co-crystal of celecoxib and nicotinamide.

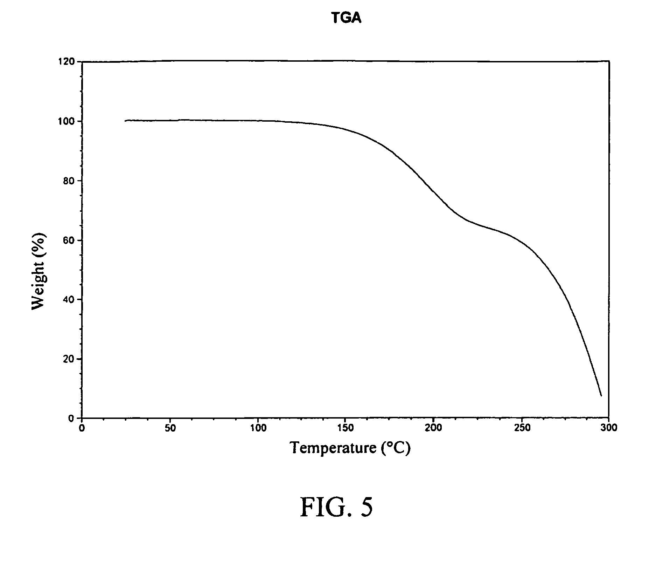

Fig. 5 -TGA thermogram for a co-crystal of celecoxib and nicotinamide.

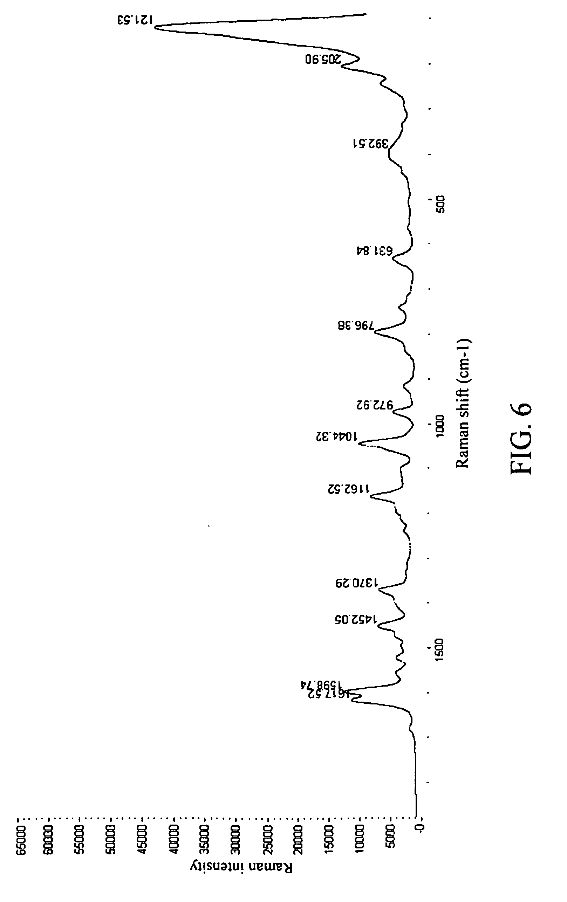

Fig. 6 -Raman spectrum for a co-crystal of celecoxib and nicotinamide.

The term "co-crystal" as used herein means a crystalline material comprised of two or more unique solids at room temperature, each containing distinctive physical characteristics, such as structure, melting point and heats of fusion.

Pharmaceutical compositions are discussed in detail below and may further comprise a pharmaceutically-acceptable diluent, excipient or carrier.

Pharmaceutical compositions of the invention optionally comprise one or more pharmaceutically acceptable carriers or diluents as excipients. Suitable carriers or diluents illustratively include, but are not limited to, either individually or in combination, lactose, including anhydrous lactose and lactose monohydrate; starches, including directly compressible starch and hydrolysed starches (e. g., Celutab and Emdex) ; marmitol; sorbitol; xylitol; dextrose (e. g., Cerelose 2000) and dextrose monohydrate; dibasic calcium phosphate dihydrate; sucrose-based diluents; confectioner's sugar; monobasic calcium sulfate monohydrate; calcium sulfate dihydrate; granular calcium lactate trihydrate; dextrates; inositol; hydrolyzed cereal solids; amylose; celluloses including microcrystalline cellulose, food grade sources of alpha-and amorphous cellulose (e. g. , Rexcel J), powdered cellulose, hydroxypropylcellulose (HPC) and hydroxypropylmethylcellulose (HPMC); calcium carbonate; glycine; bentonite; block co-polymers; polyvinylpyrrolidone; and the like. Such carriers or diluents, if present, constitute in total about 5% to about 99%, preferably about 10% to about 85%, and more preferably about 20% to about 80%, of the total weight of the composition. The carriers, carriers, diluent, or diluents selected preferably exhibit suitable flow properties and, where tablets are desired, compressibility. Lactose, mannitol, dibasic sodium phosphate, and microcrystalline cellulose (particularly Avicel PH microcrystalline cellulose such as Avicel PH 101), either individually or in combination, are preferred diluents. These diluents are chemically compatible with many co-crystals described herein. The use of extragranular microcrystalline cellulose (that is, microcrystalline cellulose added to a granulated composition) can be used to improve hardness (for tablets) and/or disintegration time. Lactose, especially lactose monohydrate, is particularly preferred. Lactose typically provides compositions having suitable release rates of co-crystals, stability, pre- compression flowability, and/or drying properties at a relatively low diluent cost. It provides a high density substrate that aids densification during granulation (where wet granulation is employed) and therefore improves blend flow properties and tablet properties.

Pharmaceutical compositions of the invention optionally comprise one or more pharmaceutically acceptable disintegrants as excipients, particularly for tablet formulations. Suitable disintegrants include, but are not limited to, either individually or in combination, starches, including sodium starch glycolate (e. g., Explotab of PenWest) and pregelatinized corn starches (e. g., National 1551 of National Starch and Chemical Company, National 1550, and Colorcon 1500), clays (e. g., Veegum HV of R. T. Vanderbilt), celluloses such as purified cellulose, microcrystalline cellulose, methylcellulose, carboxymethylcellulose and sodium carboxymethylcellulose, croscarmellose sodium (e. g., Ac-Di-Sol of FMC), alginates, crospovidone, and gums such as agar, guar, locust bean, karaya, pectin and tragacanth gums. Disintegrants may be added at any suitable step during the preparation of the composition, particularly prior to granulation or during a lubrication step prior to compression. Such disintegrants, if present, constitute in total about 0. 2% to about 30%, preferably about 0. 2% to about 10%, and more preferably about 0. 2% to about 5%, of the total weight of the composition. Croscarmellose sodium is a preferred disintegrant for tablet or capsule disintegration, and, if present, preferably constitutes about 0. 2% to about 10%, more preferably about 0. 2% to about 7%, and still more preferably about 0.2% to about 5%, of the total weight of the composition. Croscarmellose sodium confers superior intragranular disintegration capabilities to granulated pharmaceutical compositions of the present invention.

Pharmaceutical compositions of the invention optionally comprise one or more pharmaceutically acceptable binding agents or adhesives as excipients, particularly for tablet formulations. Such binding agents and adhesives preferably impart sufficient cohesion to the powder being tableted to allow for normal processing operations such as sizing, lubrication, compression and packaging, but still allow the tablet to disintegrate and the composition to be absorbed upon ingestion. Such binding agents may also prevent or inhibit crystallization or recrystallization of a co- crystal of the present invention once the salt has been dissolved in a solution. Suitable binding agents and adhesives include, but are not limited to, either individually or in combination, acacia; tragacanth; sucrose; gelatin; glucose; starches such as, but not limited to, pregelatinized starches (e. g., National 1511 and National 1500); celluloses such as, but not limited to, methylcellulose and carmellose sodium (e. g., Lose) ; alginic acid and salts of alginic acid; magnesium aluminum silicate; PEG; guar gum; polysaccharide acids; bentonites; povidone, for example povidone K-15, K-30 and K-29/32; polymethacrylates; HPMC ; hydroxypropylcellulose (e. g., KlucelTM of Aqualon) ; and ethylcellulose (e. g., Ethocel of the Dow Chemical Company). Such binding agents and/or adhesives, if present, constitute in total about 0.5% to about 25%, preferably about 0.75% to about 15%, and more preferably about 1% to about 10%, of the total weight of the pharmaceutical composition. Many of the binding agents are polymers comprising amide, ester, ether, alcohol or ketone groups and, as such, are preferably included in pharmaceutical compositions of the present invention. Polyvinylpyrrolidones such as povidone K-30 are especially preferred. Polymeric binding agents can have varying molecular weight, degrees of crosslinking, and grades of polymer. Polymeric binding agents can also be copolymers, such as block co-polymers that contain mixtures of ethylene oxide and propylene oxide units. Variation in these units/ratios in a given polymer affects properties and performance. Examples of block co-polymers with varying compositions of block units are Poloxamer 188 and Poloxamer 237 (BASF Corporation).

Pharmaceutical compositions of the invention optionally comprise one or more pharmaceutical acceptable wetting agents as excipients. Such wetting agents are preferably selected to maintain the co-crystal in close association with water, a condition that is believed to improve bioavailability of the composition, Such wetting agents can also be useful in solubilizing or increasing the solubility of co-crystals. Non-limiting examples of surfactants that can be used as wetting agents in pharmaceutical compositions of the invention include quaternary ammonium compounds, for example benzalkonium chloride, benzethonium chloride and cetylpyridinium chloride, dioctyl sodium sulfosuccinate, polyoxyethylene alkylphenyl ethers, for example nonoxynol 9, nonoxynol 10, and degrees Ctoxynol 9, poloxamers (polyoxyethylene and polyoxypropylene block copolymers), polyoxyethylene fatty acid glycerides and oils, for example polyoxyethylene (8) caprylic/capric mono-and diglycerides (e, g., LabrasolTM of Gattefosse), polyoxyethylene (35) castor oil and polyoxyethylene (40) hydrogenated castor oil; polyoxyethylene alkyl ethers, for example polyoxyethylene (20) cetostearyl ether, polyoxyethylene fatty acid esters, for example polyoxyethylene (40) stearate, polyoxyethylene sorbitan esters, for example polysorbate 20 and polysorbate 80 (e. g., Tween 80 of ICI), propylene glycol fatty acid esters, for example propylene glycol laurate (e. g., LauroglycolTM of Gattefosse), sodium lauryl sulfate, fatty acids and salts thereof, for example oleic acid, sodium oleate and triethanolamine oleate, glyceryl fatty acid esters, for example glyceryl monostearate, sorbitan esters, for example sorbitan monolaurate, sorbitan monooleate, sorbitan monopalmitate and sorbitan monostearate, tyloxapol, and mixtures thereof. Such wetting agents, if present, constitute in total about 0.25% to about 15%, preferably about 0.4% to about 10%, and more preferably about 0.5% to about 5%, of the total weight of the pharmaceutical composition. Wetting agents that are anionic surfactants are preferred. Sodium lauryl sulfate is a particularly preferred wetting agent. Sodium lauryl sulfate, if present, constitutes about 0.25% to about 7%, more preferably about 0,4% to about 4%, and still more preferably about 0.5% to about 2%, of the total weight of the pharmaceutical composition.

Pharmaceutical compositions of the invention optionally comprise one or more pharmaceutically acceptable lubricants (including anti-adherents and/or glidants) as excipients. Suitable lubricants include, but are not limited to, either individually or in combination, glyceryl behapate (e. g., Compritol 888 of Gattefosse) ; stearic acid and salts thereof, including magnesium, calcium and sodium stearates; hydrogenated vegetable oils (e, g., SterotexT of Abitec) ; colloidal silica; talc ; waxes; boric acid; sodium benzoate; sodium acetate; sodium fumarate; sodium chloride ; DL-leucine; PEG (e. g., Carbowarm 4000 and Carbowaxrin 6000 of the Dow Chemical Company); sodium oleate ; sodium lauryl sulfate; and magnesium lauryl sulfate. Such lubricants, if present, constitute in total about 0. 1% to about 10%, preferably about 0.2% to about 8%, and more preferably about 0. 25% to about 5%, of the total weight of the pharmaceutical composition. Magnesium stearate is a preferred lubricant used, for example, to reduce friction between the equipment and granulated mixture during compression of tablet formulations.

Suitable anti-adherents include, but are not limited to, talc, cornstarch, DL- leucine, sodium lauryl sulfate and metallic stearates. Talc is a preferred anti-adherent or glidant used, for example, to reduce formulation sticking to equipment surfaces and also to reduce static in the blend. Talc, if present, constitutes about 0. 1% to about 10%, more preferably about 0.25% to about 5%, and still more preferably about 0.5% to about 2%, of the total weight of the pharmaceutical composition.

Glidants can be used to promote powder flow of a solid formulation. Suitable glidants include, but are not limited to, colloidal silicon dioxide, starch, talc, tribasic calcium phosphate, powdered cellulose and magnesium trisilicate. Colloidal silicon dioxide is particularly preferred.

Other excipients such as colorants, flavors and sweeteners are known in the pharmaceutical art and can be used in pharmaceutical compositions of the present invention. Tablets can be coated, for example with an enteric coating, or uncoated.

Compositions of the invention can further comprise, for example, buffering agents.

Optionally, one or more effervescent agents can be used as disintegrants and/or to enhance organoleptic properties of pharmaceutical compositions of the invention. When present in pharmaceutical compositions of the invention to promote dosage form disintegration, one or more effervescent agents are preferably present in a total amount of about 30% to about 75%, and preferably about 45% to about 70%, for example about 60%, by weight of the pharmaceutical composition. According to a particularly preferred embodiment of the invention, an effervescent agent, present in a solid dosage form in an amount less than that effective to promote disintegration of the dosage form, provides improved dispersion of the API in an aqueous medium. Without being bound by theory, it is believed that the effervescent agent is effective to accelerate dispersion of the API from the dosage form in the gastrointestinal tract, thereby further enhancing absorption and rapid onset of therapeutic effect. When present in a pharmaceutical composition of the invention to promote intragastrointestinal dispersion but not to enhance disintegration, an effervescent agent is preferably present in an amount of about 1% to about 20%, more preferably about 2.5% to about 15%, and still more preferably about 5% to about 10%, by weight of the pharmaceutical composition. An "effervescent agent" herein is an agent comprising one or more compounds which, acting together or individually, evolve a gas on contact with water. The gas evolved is generally oxygen or, most commonly, carbon dioxide. Preferred effervescent agents comprise an acid and a base that react in the presence of water to generate carbon dioxide gas. Preferably, the base comprises an alkali metal or alkaline earth metal carbonate or bicarbonate and the acid comprises an aliphatic carboxylic acid. Non-limiting examples of suitable bases as components of effervescent agents useful in the invention include carbonate salts (e. g. , calcium carbonate), bicarbonate salts (e. g. , sodium bicarbonate), sesquicarbonate salts, and mixtures thereof. Calcium carbonate is a preferred base. Non-limiting examples of suitable acids as components of effervescent agents and/or solid organic acids useful in the invention include citric acid, tartaric acid (as D-, L-, or D/L-tartaric acid), malic acid (as D-, L-, or DL-malic acid), maleic acid, fumaric acid, adipic acid, succinic acid, acid anhydrides of such acids, acid salts of such acids, and mixtures thereof. Citric acid is a preferred acid. In a preferred embodiment of the invention, where the effervescent agent comprises an acid and a base, the weight ratio of the acid to the base is about 1: 100 to about 100; 1, more preferably about 1: 50 to about 50: 1, and still more preferably about 1: 10 to about 10: 1. In a further preferred embodiment of the invention, where the effervescent agent comprises an acid and a base, the ratio of the acid to the base is approximately stoichiometric.

Excipients which solubilize APIs typically have both hydrophilic and hydrophobic regions, or are preferably amphiphilic or have amphiphilic regions. One type of amphiphilic or partially-amphiphilic excipient comprises an amphiphilic polymer or is an amphiphilic polymer. A specific amphiphilic polymer is a polyalkylene glycol, which is commonly comprised of ethylene glycol and/or propylene glycol subunits. Such polyalkylene glycols can be esterified at their termini by a carboxylic acid, ester, acid anhyride or other suitable moiety. Examples of such excipients include poloxamers (symmetric block copolymers of ethylene glycol and propylene glycol; e. g., poloxamer 237), polyalkyene glycolated esters of tocopherol (including esters formed from a di-or multi-functional carboxylic acid; e. g., d-alpha-tocopherol polyethylene glycol-1000 succinate), and macrogolglycerides (formed by alcoholysis of an oil and esterification of a polyalkylene glycol to produce a mixture of mono-, di-and tri-glycerides and mono-and di-esters; e. g., stearoyl macrogol-32 glycerides). Such pharmaceutical compositions are advantageously administered orally.

Pharmaceutical compositions of the present invention can comprise about 10 % to about 50 %, about 25 % to about 50 %, about 30 % to about 45 %, or about 30 % to about 35 % by weight of a co-crystal; about 10 % to about 50 %, about 25 % to about 50 %, about 30 % to about 45 %, or about 30 % to about 35 % by weight of an excipient which inhibits crystallization in aqueous solution, in simulated gastric fluid, or in simulated intestinal fluid; and about 5 % to about 50 %, about 10 % to about 40 %, about 15 % to about 35 %, or about 30 % to about 35 % by weight of a binding agent. In one example, the weight ratio of the co-crystal to the excipient which inhibits crystallization to binding agent is about I to 1 to 1.

Solid dosage forms of the invention can be prepared by any suitable process, not limited to processes described herein. An illustrative process comprises (a) a step of blending the API of the invention with one or more excipients to form a blend, and (b) a step of tableting or encapsulating the blend to form tablets or capsules, respectively. In a preferred process, solid dosage forms are prepared by a process comprising (a) a step of blending a co-crystal of the invention with one or more excipients to form a blend, (b) a step of granulating the blend to form a granulate, and (c) a step of tableting or encapsulating the blend to form tablets or capsules respectively. Step (b) can be accomplished by any dry or wet granulation technique known in the art, but is preferably a dry granulation step. A salt of the present invention is advantageously granulated to form particles of about I micrometer to about 100 micrometer, about 5 micrometer to about 50 micrometer, or about 10 micrometer to about 25 micrometer. One or more diluents, one or more disintegrants and one or more binding agents are preferably added, for example in the blending step, a wetting agent can optionally be added, for example in the granulating step, and one or more disintegrants are preferably added after granulating but before tableting or encapsulating. A lubricant is preferably added before tableting. Blending and granulating can be performed independently under low or high shear. A process is preferably selected that forms a granulate that is uniform in API content, that readily disintegrates, that flows with sufficient ease so that weight variation can be reliably controlled during capsule filling or tableting, and that is dense enough in bulk so that a batch can be processed in the selected equipment and individual doses fit into the specified capsules or tablet dies. In an alternative embodiment, solid dosage forms are prepared by a process that includes a spray drying step, wherein an API is suspended with one or more excipients in one or more sprayable liquids, preferably a non-protic (e. g. , non-aqueous or non-alcoholic) sprayable liquid, and then is rapidly spray dried over a current of warm air.

A granulate or spray dried powder resulting from any of the above illustrative processes can be compressed or molded to prepare tablets or encapsulated to prepare capsules. Conventional tableting and encapsulation techniques known in the art can be employed. Where coated tablets are desired, conventional coating techniques are suitable. Excipients for tablet compositions of the invention are preferably selected to provide a disintegration time of less than about 30 minutes, preferably about 25 minutes or less, more preferably about 20 minutes or less, and still more preferably about 15 minutes or less, in a standard disintegration assay.

Pharmaceutically acceptable co-crystals can be administered by controlled-or delayed-release means. Controlled-release pharmaceutical products have a common goal of improving drug therapy over that achieved by their non-controlled release counterparts. Ideally, the use of an optimally designed controlled-release preparation in medical treatment is characterized by a minimum of drug substance being employed to cure or control the condition in a minimum amount of time. Advantages of controlled-release formulations include:
1) extended activity of the drug;
2) reduced dosage frequency;
3) increased patient compliance;
4) usage of less total drug;
5) reduction in local or systemic side effects;
6) minimization of drug accumulation;
7) reduction in blood level fluctuations;
8) improvement in efficacy of treatment;
9) reduction of potentiation or loss of drug activity; and
10) improvement in speed of control of diseases or conditions.

Kim, Chemg-ju, Controlled Release Dosage Form Design, 2 (Technomic Publishing, ancaster, Pa.: 2000).

Conventional dosage forms generally provide rapid or immediate drug release from the formulation. Depending on the pharmacology and pharmacokinetics of the drug, use of conventional dosage forms can lead to wide fluctuations in the concentrations of the drug in a patient's blood and other tissues. These fluctuations can impact a number of parameters, such as dose frequency, onset of action, duration of efficacy, maintenance of therapeutic blood levels, toxicity, side effects, and the like. Advantageously, controlled-release formulations can be used to control a drug's onset of action, duration of action, plasma levels within the therapeutic window, and peak blood levels. In particular, controlled-or extended-release dosage forms or formulations can be used to ensure that the maximum effectiveness of a drug is achieved while minimizing potential adverse effects and safety concerns, which can occur both from under dosing a drug (i.e., going below the minimum therapeutic levels) as well as exceeding the toxicity level for the drug. Most controlled-release formulations are designed to initially release an amount of drug (active ingredient) that promptly produces the desired therapeutic effect, and gradually and continually release other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, ionic strength, osmotic pressure, temperature, enzymes, water, and other physiological conditions or compounds.

A variety of known controlled-or extended-release dosage forms, formulations, and devices can be adapted for use with the co-crystals and compositions of the invention. Examples include, but are not limited to, those described in U. S. Pat. Nos. 3845770; 3916899; 3536809;3598123; 4008719; 674533;5059595; 591767; 5120548;5073543; 5639476; 5354556; 5733566 ; and 6365185. These dosage forms can be used to provide slow or controlled-release of one or more active ingredients using, for example, hydroxypropylmethyl cellulose, other polymer matrices, gels, permeable membranes, osmotic systems such as OROS (Alza Corporation, Mountain View, Calif. USA)), multilayer coatings, microparticles, liposomes, or microspheres or a combination thereof to provide the desired release profile in varying proportions. Additionally, ion exchange materials can be used to prepare immobilized, adsorbed co-crystals and thus effect controlled delivery of the drug. Examples of specific anion exchangers include, but are not limited to, Duolite A568 and Duolite AP143 (Rohm & Haas, Spring House, PA. USA).

One embodiment of the invention encompasses a unit dosage form which comprises a pharmaceutically acceptable co-crystal, or a solvate, hydrate, dehydrate, anhydrous, or amorphous form thereof, and one or more pharmaceutically acceptable excipients or diluents, wherein the pharmaceutical composition or dosage form is formulated for controlled-release. Specific dosage forms utilize an osmotic drug delivery system.

A particular and well-known osmotic drug delivery system is referred to as OROS (Alza Corporation, Mountain View, Calif. USA). This technology can readily be adapted for the delivery of compounds and compositions of the invention. Various aspects of the technology are disclosed in U. S. Pat. Nos. 6375978, 6368626; 6342249; 6333050; 6287295; 6283953; 6270787; 6245357; and 6132420. Specific adaptations of OROS that can be used to administer compounds and compositions of the invention include, but are not limited to, the OROS Push- Pull, Delayed Push-Pull, Multi-Layer Push-Pull, and Push-Stick Systems, all of which are well known see, e. g. http://www.alza.com. Additional OROS systems that can be used for the controlled oral delivery of compounds and compositions of the invention include OROS -CT and L-OROS. Id.; see also, Delivery Times, vol. II, issue II (Alza Corporation).

Conventional OROS oral dosage forms are made by compressing a drug powder (e. g. co-crystal) into a hard tablet, coating the tablet with cellulose derivatives to form a semipermeable membrane, and then drilling an orifice in the coating (e. g., with a laser). Kim, Chemgju. Controlled Release Dosage Form, Design, 231-238 (Technomic Publishing, Lancaster, Pa.: 2000). The advantage of such dosage forms is that the delivery rate of the drug is not influenced by physiological or experimental conditions. Even a drug with a pH-dependent solubility can be delivered at a constant rate regardless of the pH of the delivery medium. But because these advantages are provided by a build-up of osmotic pressure within the dosage form after administration, conventional OROS drug delivery systems cannot be used to effectively deliver drugs with low water solubility, Id. at 234. Because co-crystals of this invention can be far more soluble in water than the API itself, they are well suited for osmotic-based delivery to patients.

A specific dosage form of the invention comprises: a wall defining a cavity, the wall having an exit orifice formed or formable therein and at least a portion of the wall being semipermeable; an expandable layer located within the cavity remote from the exit orifice and in fluid communication with the semipermeable portion of the wall; a dry or substantially dry state drug layer located within the cavity adjacent to the exit orifice and in direct or indirect contacting relationship with the expandable layer; and a flow-promoting layer interposed between the inner surface of the wall and at least the external surface of the drug layer located within the cavity, wherein the drug layer comprises a co-crystal, or a solvate, hydrate, dehydrate, anhydrous, or amorphous form thereof, see U. S. Pat. No. 6,368, 626.

Another specific dosage form of the invention comprises: a wall defining a cavity, the wall having an exit orifice formed or formable therein and at least a portion of the wall being semipermeable; an expandable layer located within the cavity remote from the exit orifice and in fluid communication with the semipermeable portion of the wall; a drug layer located within the cavity adjacent the exit orifice and in direct or indirect contacting relationship with the expandable layer; the drug layer comprising a liquid, active agent formulations absorbed in porous particles, the porous particles being adapted to resist compaction forces sufficient to form a compacted drug layer without significant exudation of the liquid, active agent formulation, the dosage form optionally having a placebo layer between the exit orifice and the drug layer, wherein the active agent formulation comprises a co-crystal, or a solvate, hydrate, dehydrate, anhydrous, or amorphous form thereof, see U. S. Pat. No. 6342249.

### EXEMPLIFICATION

### Crystallization from solution

Co-crystals may be obtained by dissolving the separate components in a solvent and adding one to the other. The co-crystal may then precipitate or crystallize as the solvent mixture is evaporated slowly. The co-crystal may also be obtained by dissolving the two components in the same solvent or a mixture of solvents.

### Analytical Methods

The co-crystals of the present invention can be characterized e.g., by the TGA or DSC data or by any one, any two, any three, any four, any five, any six, any seven, any eight, any nine, any ten, or any single integer number of PXIID 2-theta angle peaks or Raman shift peaks listed herein or disclosed in a figure, or by single crystal x-ray diffraction data.

### Procedure for DSC analysis

DSC analysis of the samples was performed using a Q1 000 Differential Scanning Calorimeter (TA Instruments, New Castle, DE, U.S.A.), which uses Advantage for QW-Series, version 1.0.0.78, Thermal Advantage Release 2.0 (⁸2001 TA Instruments- Water LLC). In addition, the analysis software used was Universal Analysis 2000 for Windows 95/95/2000/NT, version 3.1 E; Build 3.1.0.40 (⁸2001 TA Instruments- Water LLC).

For the DSC analysis, the purge gas used was dry nitrogen, the reference material was an empty aluminum pan that was crimped, and the sample purge was 50 mL/minute.

DSC analysis of the sample was performed by placing ≤ 2 mg of sample in an aluminum pan with a crimped pan closure. The starting temperature was typically 20°C with a heating rate of 10°C/minute, and the ending temperature was 300°C. Unless otherwise indicated, all reported transitions are as stated ± 1.0°C.

### Procedure for TGA analysis

TGA analysis of samples was performed using a Q500 Thermogravimetric Analyzer (TA Instruments, New Castle, DE, U.S.A.), which uses Advantage for QW-Series, version 1.0.0.78, Thermal Advantage Release 2.0 (⁸2001 TA Instruments-Water LLC). In addition, the analysis software used was Universal Analysis 2000 for Windows 95/95/2000/not, version 3.1E; Build 3.1.0.40 (⁸2001 TA Instrunlents- Water LLC).

For all of the TGA experiments, the purge gas used was dry nitrogen, the balance purge was 40 mL/minute N₂, and the sample purge was 60 mL/minute N₂.

TGA of the sample was performed by placing ≤ 2 mg of sample in a platinum pan. The starting temperature was typically 20°C with a heating rate of 10°C/minute, and the ending temperature was 300°C.

### Procedure for PXRD analysis

A powder X-ray diffraction pattern for the samples was obtained using a D/Max Rapid, Contact (Rigaku/MSC, The Woodlands, TX, U.S.A.), which uses as its control software PINT Rapid Control software, Rigaku Rapid/XRD, version 1.0.0 (⁸1999 Rigaku Co.). In addition, the analysis software used were RINT Rapid display software, version 1.18 (Rigaku/MSC), and JADE XRD Pattern Processing, versions 5.0 and 6.0 (⁸1995-2002, Materials Data, Inc.).

For the PXRD analysis, the acquisition parameters were as follows: source was Cu with a K line at 1,5406Å; x-y stage was manual; collimator size was 0.3 or 0.8 mm; capillary tube (Charles Supper Company, Natick, MA, U. S.A.) was 0.3 mm ID; reflection mode was used; the power to the X- ray tube was 46 kV; the current to the X-ray tube was 40 mA; the omega-axis was oscillating in a range of 0-5° at a speed of 1°/minute; the phi-axis was spinning at an angle of 360° at a speed of 2°/second; 0.3 or 0.8 mm collimator; the collection time was 60 minutes; the temperature was room temperature; and the heater was not used. The sample was presented to the X-ray source in a boron rich glass capillary.

In addition, the analysis parameters were as follows: the integration 2-theta range was 2-40 or 60°; the integration chi range was 0-360°; the number of chi segments was 1; the step size used was 0.02; the integration utility was cylint; normalization was used; dark counts were 8, omega offset was 180, and chi and phi offsets were 0,

The relative intensity of peaks in a diffractogram is not necessarily a limitation of the PXRD pattern because peak intensity can vary from sample to sample, e.g., due to 1 crystalline impurities. Further, the angles of each peak can vary by about ± 0.1 preferably ±0.05. The entire pattern or most of the pattern peaks may also shift by about ± 0.1 degree due to differences in calibration, settings, and other variations from instrument to instrument and from operator to operator.

### Procedure for Raman Acquisitions Filtering and Binning

### Acquisition

The sample was either left in the glass vial in which it was processed or an aliquot of the sample was transferred to a glass slide. The glass vial or slide was positioned in the sample chamber. The measurement was made using an Almega^{™} Dispersive Raman (Almega Dispersive Raman, Thermo-Nicolet, 5225 Verona Road, Madison, WI 53711-4495) system fitted with a 785nm laser source. The sample was manually brought into focus using the microscope portion of the apparatus with a 10x power objective (unless otherwise noted), thus directing the laser onto the surface of the sample. The spectrum was acquired using the parameters outlined in Table A. (Exposure times and number of exposures may vary, changes to parameters will be indicated for each acquisition.)

### Filtering and Binding

Each spectrum in a set was filtered using a matched filter of feature size 25 to remove background signals, including glass contributions and sample fluorescence. This is particularly important as large background signal or fluorescence limit the ability to accurately pick and assign peak positions in the subsequent steps of the binning process. Filtered spectra were binned using the peak pick and bin algorithm with the parameters given in Table B. The sorted cluster diagrams for each sample set and the corresponding cluster assignments for each spectral file were used to identify groups of samples with similar spectra, which was used to identify samples for secondary analyses.

**Table A. Raman Spectral acquisition parameters**

| **Parameter** | **Setting Used** |
|---|---|
| Exposure time (s) | 2.01 I |
| Number of exposures | 10 |
| Laser source wavelength (nm) | 785 |
| Laser power (%) | 100 |
| Aperture shape | pin hole |
| Aperture size (µm) | 100 |
| Spectral range | 104-3428 |
| Grating position | Single |
| Temperature at acquisition | 24.0°C |

**Table B. Raman Filtering and Binning Parameters**

| **Parameter** | | **Setting Used** |
|---|---|---|
| *Filtering Parameters* | | |
| | Filter type | Matched |
| | Filter size | 25 |
| *QC Parameter* | | |
| | Peak Height Threshold | 1000 |
| | Region for noise test (cm⁻¹) | 0-10000 |
| | RMS noise threshold | 10000 |
| Automatically eliminate | | Yes |
| failed spectra | | |
| *Region of Interest* | | |
| | Include (cm⁻¹) | 104-3428 |
| | Exclude region I (cm⁻¹) | |
| | Exclude region II (cm⁻¹) | |
| | Exclude region III (cm⁻¹) | |
| | Exclude region IV (cm⁻¹) | |
| *Peak Pick Parameters* | | |
| | Peak Pick Sensitivity | Variable |
| | Peak Pick Threshold | 100 |
| Peak Comparison Parameters | | |
| | Peak Window (cm⁻¹) | 2 |
| Analysis Parameters | | |
| | Number of clusters | Variable |

### Procedure for Single Crystal X-Ray Diffraction

Single crystal x-ray data were collected on a Bruker SMART-APEX CCD diffractometer (M. J, Zawarotko, Department of Chemistry, University of South Florida). Lattice parameters were determined from least squares analysis. Reflection data was integrated using the program SAINT. The structure was solved by direct methods and refined by full matrix least squares using the program SHELXTL (Sheldrick, G. M. SHELXTL, Release 5.03, Siemens Analytical X- ray Instruments Inc. Madison, WI).

### Example

Celccoxib : nicotinamide co-crystals were prepared. Celecoxib (100 mg, 0.26 mmol) and nicotinamide (32.0 mg, 0.26 mmol) were each dissolved in acetone (2 mL). The two solutions were mixed and the resulting mixture was allowed to evaporate slowly overnight. The precipitated solid was collected and characterized.

| | |
|---|---|
| **PXRD:** | 3.77, 7.56, 9.63, 14.76, 15.21, 16.01, 17.78, 18.68, 19.31, 20.44, 21.19, 22.10; see also Fig. 3. |
| **DSC:** | two endothermic transitions at 117.2 and 118.8°C and a sharp endotherm at 129.7°C; see also Fig. 4. |
| **TGA:** | Decomposition begins -150°C; see also Fig. 5. |
| **Raman:** | 1617.5, 1598.7, 1452.1, 1370.3, 1162.5, 1044.3, 972.9, 796.4, 631.8, 392.5, 205.9; see also Fig. 6. |
| **Method:** | Slow evaporation of a 1:1 solution from acetone |

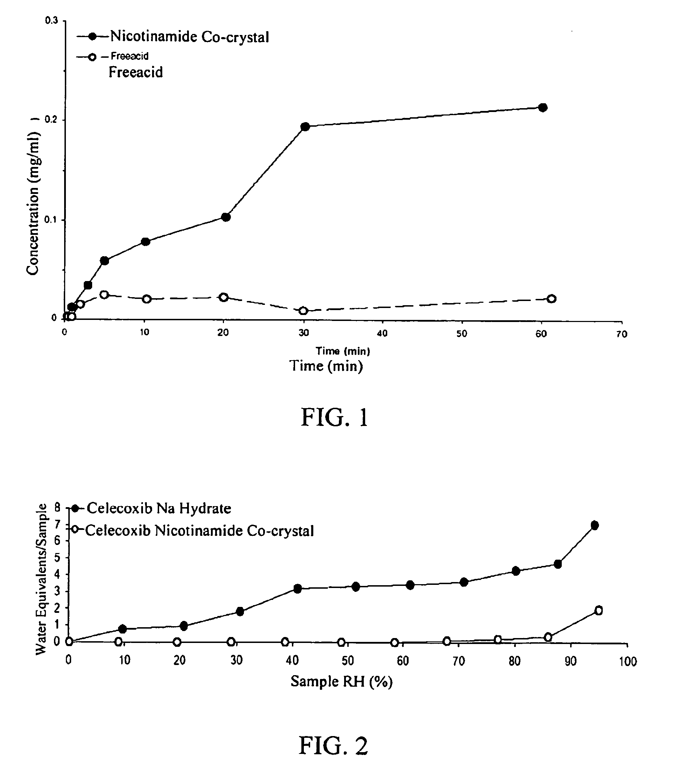

A modified dissolution profile is shown in Fig 1 and an improved hygroscopicity profile is shown in Fig. 2.

## Claims

1. A co-crystal comprising celecoxib and nicotinamide.

2. A pharmaceutical composition comprising the co-crystal of claim 1 and a pharmaceutically acceptable diluent, excipient or carrier.

## Patentansprüche

1. Mischkristall, umfassend Celecoxib und Nicotinamid.

2. Pharmazeutische Zubereitung, umfassend den Misclvkristall nach Anspruch 1 und ein pharmazeutisch akzeptables Verdünnungsmittel, Corrigens oder einen pharmazeutisch akzeptablen Träger.

## Revendications

1. Co-cristal comprenant le celecoxib et le nicotinamide.

2. Composition pharmaceutique comprenant le co-cristal de la revendication 1 et un diluant pharmaceutiquement acceptable, excipient ou véhicule.
